# EUROPEAN PATENT SPECIFICATION

(11) **EP 1 706 473 B1**
(45) Date of publication and mention of the grant of the patent: **20.02.2008**
(21) Application number: 04811763.4
(22) Date of filing: 22.11.2004
(51) Int. Cl.: C11D 3/00

(54) **NONIONIC SURFACTANT CONTAINING COMPOSITIONS FOR CLEANING CONTACT LENSES**
NICHTIONISCHES TENSID ENTHALTENDE ZUSAMMENSETZUNGEN ZUR REINIGUNG VON KONTAKTLINSEN
COMPOSITIONS CONTENANT UN TENSIO-ACTIF NON IONIQUE POUR LE NETTOYAGE DES LENTILLES DE CONTACT

(30) Priority: 01.12.2003 US 724679
(43) Date of publication of application: 04.10.2006
(73) Proprietor: BAUSCH & LOMB INCORPORATED, Rochester, New York 14604 (US)
(72) Inventor: XIA, Earning, Penfield, NY 14526 (US); DOBIE, Alyce, K., Williamson, NY 14589 (US); SALMONE, Joseph, C., Fairport, NY 14450 (US); HU, Zhenze, Pittsford, NY 14534 (US); AMMON, Daniel, M., Jr., Rochester, NY 14609 (US)
(74) Representative: Maiwald, Walter
(86) International application number: PCT/US2004/039102
(87) International publication number: WO 2005/054418

(56) References cited:
- EP-A- 0 255 041
- EP-A- 0 439 429
- EP-A- 0 524 150
- WO-A-02/26922
- WO-A-95/01414
- US-A- 4 046 706
- US-A- 4 504 405
- US-A1- 2002 141 899
- US-A1- 2003 119 684

## Description

### Field of the Invention:

The present invention relates to compositions and methods for cleaning and disinfecting contact lenses. Compositions of the present invention are particularly effective in the removal of lipid deposits from the surfaces of contact lenses, thus rendering the contact lenses more comfortable for the contact lens wearer. Additionally, the subject compositions provide a prophylactic effect in preventing lipid deposits from forming on a contact lens having been soaked in the composition prior to the lens being worn.

### Background of the Invention:

Conventionally, contact lenses have been classified into water-nonabsorptive contact lenses and water-absorptive contact lenses, and classified into hard contact lenses and soft contact lenses. Both hard and soft contact lenses may develop deposits or a stain of lipids derived from tears while the lens is worn in the eye. Such lipid stains may cause a deterioration in the comfort of a lens during wear or cause eye problems such as blurred eyesight or congestion of the cornea. Accordingly, it is essential to apply a cleaning treatment to a contact lens in order to safely and comfortably use contact lenses every day.

To effectively clean contact lenses, solutions formulated for cleaning contact lenses having cleaning or removal effect over one or more stains are typically used. Solutions formulated for cleaning contact lenses may include therein a surfactant useful as a cleaning component. Contact lens cleaning solutions incorporating nonionic surfactants such as a polyoxyalkylene block copolymer such as a polyoxyethylene-polyoxypropylene block copolymer or a derivative thereof are known.

However, cleaning solutions for contact lenses containing nonionic surfactants may risk causing eye irritation. Great importance is attached to the safety and comfort of lens care solutions, thus requiring the concentration of cleaning surfactants, if any in the solution, to be maintained as low as possible. Experience shows that conventional cleaning solutions for contact lenses containing cleaning surfactants at low concentrations to avoid eye discomfort or irritation, lack adequate cleaning power or lipid-solubilizing power. As a result, cleaning treatments of contact lenses using a low concentration surfactant cleaning solution, tend to allow lipid stains to remain and accumulate on the contact lens, potentially being harmful to the eye.

U.S. Patent Number 5,500,144 (Potini et al.), discloses compositions for the care of contact lenses including a silicone polymer containing an alkyleneoxide side chain. Included in the silicone polymer compositions are nonionic surface active agents having good cleaning activity, such as polyoxyethylene, polyoxypropylene block copolymers having hydrophilic/lipophilic balances (HLBs) of generally about 12 to about 18, as opposed to other poloxamers that may also be employed in the compositions as primary cleaning agents having HLBs of at least about 18.

U.S. Patent Number 6,417,144 (Tsuzuki et al.), discloses a solution for contact lenses comprising the combination of an amino acid type cationic surfactant and at least one nonionic surfactant with an HLB above 18 whereby cleaning powers are synergistically increased over the use of either an amino acid type cationic surfactant or a nonionic surfactant independently.

As mentioned above, nonionic surfactants are well known in the art of contact lens cleaning. However independent use of nonionic surfactants for cleaning contact lenses appear to have considerable limitations in cleaning effectiveness at low concentrations and are known to potentially cause ocular irritation at higher concentrations. Accordingly, it would be desirable to find a contact lens cleaning solution effective in removing lipid stains without causing ocular irritation.

### Summary of the Invention:

The present invention provides compositions as defined in claim 1 for removing, reducing and/or preventing lipid deposits on contact lenses. Also, methods for removing lipid deposits from surfaces of contact lenses and for preventing or reducing the amount of such deposits thereon are provided, see the claims annexed to this specification.

According to various preferred embodiments, lipid deposits can be removed from surfaces of a contact lens without manual rubbing of the lens, for example, by rinsing.

### Brief Description of the Drawings:

FIGURE 1 is a graph of lipid cleaning (absorbance at 485 nm) vs. concentration of nonionic polyether surfactant; and
FIGURE 2 is a graph illustrating the effect of solution volume on the lipid cleaning efficacy.

### Detailed Description of the Invention:

Compositions of the present invention may be used with all contact lenses such as conventional hard, soft, rigid and soft gas permeable, and silicone (including both hydrogel and non-hydrogel) lenses, but is preferably employed with soft hydrogel lenses. Such lenses are commonly prepared from hydrophilic monomers such as 2-hydroxyethyl (meth)acrylate, N-vinylpyrrolidone, glycerol (meth)acrylate, and (meth)acrylic acid. In the case of silicone hydrogel lenses, a silicone-containing monomer is copolymerized with at least one hydrophilic monomer. Such lenses absorb significant amounts of water, typically from 10 to 80 percent by weight, and especially 20 to 70 percent water.

Compositions employed in this invention are aqueous solutions. The compositions include, as an essential component, one or more nonionic polyether surfactants. Suitable nonionic polyether surfactants for use in compositions of the present invention include for example but are not limited to Pluronic P123^{™} (BASF, Mount Olive, New Jersey) having a HLB of 8, Pluronic L42^{™} (BASF) having a HLB of 8, Pluronic L62^{™} (BASF) having a HLB of 7, Pluronic L72^{™} (BASF) having a HLB of 7, Pluronic L92^{™} (BASF) having a HLB of 6, Pluronic P103^{™} (BASF) having a HLB of 9, Pluronic R 12R3^{™} (BASF) having a HLB of 7, Pluronic R 17R1^{™} (BASF) having a HLB of 3, Pluronic R 17R2^{™} (BASF) having a HLB of 6, Pluronic R 31R1T^{™} (BASF) having a HLB of 1, Pluronic R 31 R2^{™} (BASF) having a HLB of 2, Pluronic R 31 R4^{™} (BASF) having a HLB of 7, Tetronic 701^{™} (BASF) having a HLB of 3, Tetronic 702^{™} (BASF) having a HLB of 7, Tetronic 901^{™} (BASF) having a HLB of 3, Tetronic 1101^{™} (BASF) having a HLB of 2, Tetronic 1102^{™} (BASF) having a HLB of 6, Tetronic 1301^{™} (BASF) having a HLB of 2, Tetronic 1302^{™} (BASF) having a HLB of 6, Tetronic 1501^{™} (BASF) having a HLB of 1, Tetronic 1502^{™} (BASF) having a HLB of 5, Tetronic R 50R1^{™} (BASF) having a HLB of 3, Tetronic R 50R4^{™} (BASF) having a HLB of 9, Tetronic R 70R1^{™} (BASF) having a HLB of 3, Tetronic R 70R2^{™} (BASF) having a HLB of 5, Tetronic R 70R4^{™} (BASF) having a HLB of 8, Tetronic R 90R1^{™} (BASF) having a HLB of 2, Tetronic R 90R4^{™} (BASF) having a HLB of 7, Tetronic R 110R1^{™} (BASF) having a HLB of 2, Tetronic R 110R2^{™} (BASF) having a-HLB of 4, Tetronic R 110R7^{™} (BASF) having a HLB of 10, Tetronic R 130R1^{™} (BASF) having a HLB of 1, Tetronic R 130R2^{™} (BASF) having a HLB of 3, Tetronic R 150R1^{™} (BASF) having a HLB of 1, Tetronic R 150R4^{™} (BASF) having a HLB of 5 and Tetronic R 150R8^{™} (BASF) having a HLB of 11,

Relatively low HLB values less than 12 indicate a higher affinity for both hydrophobic molecules and/or surfaces, such as lipids and hydrophilic molecules. Relatively low HLB nonionic polyether surfactants have been found to significantly decrease lipid affinity to the surface of contact lenses, and are effective in removing lipids from the surface of contact lenses without mechanical or digital cleaning. Such nonionic polyether surfactants are preferably employed in compositions of the present invention in amounts ranging from about 0.1 to about 6.0 weight percent, more preferably from about 0.2 to about 5.0 weight percent to achieve cleaning efficacy.

According to various preferred embodiments of the present invention, the subject compositions are likewise suitable for disinfecting a contact lens soaked therein. In addition to water, the subject compositions also include at least one antimicrobial agent, especially a non-oxidative antimicrobial agent that derives its antimicrobial activity through a chemical or physicochemical interaction with organisms. So that the contact lenses treated with the composition may be instilled directly in the eye, i.e., without rinsing the contact lens with a separate composition, the antimicrobial agent needs to be an opthalmically acceptable antimicrobial agent.

Suitable antimicrobial agents for use in the present invention include quaternary ammonium salts which do not include significant hydrophobic portions, e.g., alkyl chains comprising more than six carbon atoms. Suitable quaternary ammonium salts for use in the present invention include for example but are not limited to poly[(dimethyliminio)-2-butene-1,4-diyl chloride] and [4-tris(2-hydroxyethyl)ammonio]-2-butenyl-ω-[tris(2-hydroxyethyl)ammonio] dichloride (Chemical Abstracts Registry Number 75345-27-6) generally available as Polyquaternium 1 (Onyx Corporation, Montpelier, Vermont). Also suitable are biguanides and their salts, such as 1,1'-hexamethylene-bis[5-(2-ethylhexyl)biguanide] (Alexidine) and poly(hexamethylene biguanide) (PHMB) available from ICI Americas, Inc., Wilmington Delaware under the trade name Cosmocil CQ, benzalkonium chloride (BAK) and sorbic acid.

One or more antimicrobial agents are present in the subject compositions in an amount effective for disinfecting a contact lens, as found in conventional lens soaking and disinfecting solutions. Preferably, the antimicrobial agent will be used in a disinfecting amount or an amount from about 0.0001 to about 0.5 weight percent by volume. A disinfecting amount of an antimicrobial agent is an amount that will at least partially reduce the microorganism population in the formulations employed. Preferably, a disinfecting amount is that which will reduce the microbial burden by two log orders in four hours and more preferably by one log order in one hour. Most preferably, a disinfecting amount is an amount that will eliminate the microbial burden on a contact lens when used in the regimen for the recommended soaking time (FDA Chemical Disinfection Efficacy Test - July, 1985 Contact Lens Solution Draft Guidelines). Typically, such agents are present in concentrations ranging from about 0.00001 to about 0.5 weight percent based on volume (w/v), and more preferably, from about 0.00003 to about 0.05 weight percent.

Compositions of the present invention may also contain various other components including for example but not limited to one or more chelating and/or sequestering agents, one or more osmolarity adjusting agents, one or more surfactants, one or more buffering agents and/or one or more wetting agents.

Chelating agents, also referred to as sequestering agents, are frequently employed in conjunction with an antimicrobial agent. These agents bind heavy metal ions, which might otherwise react with the lens and/or protein deposits and collect on the lens. Chelating agents are well known in the art, and examples of preferred chelating agents include ethylenediaminetetraacetic acid (EDTA) and its salts, especially disodium EDTA. Such agents are normally employed in amounts from about 0.01 to about 2.0 weight percent, more preferably from about 0.01 to about 0.3 weight percent. Other suitable sequestering agents include gluconic acid, citric acid, tartaric acid and their salts, e.g., sodium salts.

Compositions of the present invention may be designed for a variety of osmolarities, but it is preferred that the compositions are iso-osmal with respect to eye fluids. Specifically, it is preferred that the compositions have an osmotic value of less than about 350 mOsm/kg, more preferably from about 175 to about 330 mOsm/kg, and most preferably from about 260 to about 310 mOsm/Kg. One or more osmolarity adjusting agents may be employed in the composition to obtain the desired final osmolarity. Examples of suitable osmolarity adjusting agents include, but are not limited to sodium and potassium chloride, monosaccharides such as dextrose, calcium and magnesium chloride, and low molecular weight polyols such as glycerin and propylene glycol. Typically, these agents are used individually in amounts ranging from about 0.01 to 5 weight percent and preferably, from about 0.1 to about 2 weight percent.

Compositions of the present invention have an opthalmically compatible pH, which generally will range between about 6 to about 8, and more preferably between 6.5 to 7.8, and most preferably about 7 to 7.5. One or more conventional buffers may be employed to obtain the desired pH value. Suitable buffers include for example but are not limited to borate buffers based on boric acid and/or sodium borate, phosphate buffers based on Na₂HPO₄, NaH₂PO₄ and/or KH₂PO₄, citrate buffers based on sodium or potassium citrate and/or citric acid, sodium bicarbonate, aminoalcohol buffers and combinations thereof. Generally, buffers will be used in amounts ranging from about 0.05 to about 2.5 weight percent, and preferably, from about 0.1 to about 1.5 weight percent.

The subject compositions may likewise include a wetting agent, to facilitate the composition wetting the surface of a contact lens. Within the art, the term "humectant" is also commonly used to describe these materials. A first class of wetting agents are polymer wetting agents. Examples of suitable wetting agents include for example but are not limited to poly(vinyl alcohol) (PVA), poly(N-vinylpyrrolidone) (PVP), cellulose derivatives and poly(ethylene glycol). Cellulose derivatives and PVA may be used to also increase viscosity of the composition, and offer this advantage if desired. Specific cellulose derivatives include for example but are not limited to hydroxypropyl methyl cellulose, carboxymethyl cellulose, methyl cellulose, hydroxyethyl cellulose, and cationic cellulose derivatives. As disclosed in U.S. Patent Number 6,274,133, cationic cellulosic polymers also help prevent accumulation of lipids and proteins on a hydrophilic lens surface. Such cationic cellulosic polymers include for example but are not limited to water soluble polymers commercially available under the CTFA (Cosmetic, Toiletry, and Fragrance Association) designation Polyquaternium-10, including the cationic cellulosic polymers available under the trade name UCARE® Polymers from Amerchol Corp., Edison, New Jersey. Generally, these cationic cellulose polymers contain quaternized N,N-dimethylamino groups along the cellulosic polymer chain.

Another suitable class of wetting agents is non-polymeric wetting agents. Examples include glycerin, propylene glycol, and other non-polymeric diols and glycols.

The specific quantities of wetting agents used in the present invention will vary depending upon the application. However, the wetting agents will typically be included in an amount from about 0.01 to about 5 weight percent, preferably from about 0.1 to about 2 weight percent.

It will be understood that some constituents possess more than one functional attribute. For example, cellulose derivatives are suitable polymeric wetting agents, but are also referred to as "viscosity Increasing agents" to increase viscosity of the composition if desired. Glycerin is a suitable non-polymeric wetting agent but Is also may contribute to adjusting tonicity.

Compositions of the present invention also include at least two opthalmically acceptable nonionic surfactants. Preferred surfactants are amphoteric or nonionic surfactants. The surfactants should be soluble in the aqueous solution and non-irritating to eye tissues. The surfactants serve mainly to facilitate removal of non-proteinaceous matter on the contact lens.

Many nonionic surfactants comprise one or more chains or polymeric components having oxyalkylene (-O-R-) repeats units wherein R has 2 to 6 carbon atoms. Representative non-ionic surfactants comprise block polymers of two or more different kinds of oxyalkylene repeat units, which ratio of different repeat units determines the HLB of the surfactant. Typical HLB values for surfactants found to be suitable are in the range of 18 or above. Examples of such poloxamers are polyoxyethylene, polyoxypropylene block copolymers available under the trade name Pluronic (BASF). Poloxamines are ethylene diamine adducts of such polyoxyethylene, polyoxypropylene block copolymers available under the trade name Tetronic (BASF), including for example poloxamine 1107 (Tetronic 1107) having a molecular weight from about 7,500 to about 27,000 wherein at least 40 weight percent of said adduct is poly(oxyethylene) having a HLB of 24. Other non-ionic surfactants include for example polyethylene glycol esters of fatty acids, e.g. coconut, polysorbate, polyoxyethylene or polyoxypropylene ethers of higher alkanes (C₁₂-C₁₈), polysorbate 20 available under the trade name Tween® 20 (ICI Americas, Inc., Wilmington, Delaware), polyoxyethylene (23) lauryl ether available under the trade name Brij® 35 (ICI Americas, Inc.), polyoxyethyene (40) stearate available under the trade name Myrj® 52 (ICI Americas, Inc.), polyoxyethylene (25) propylene glycol stearate available under the trade name Atlas® G 2612 (ICI Americas, Inc.).

Another useful class of cleaning agents are the hydroxyalkylphosphonates, such as those disclosed in U.S. Patent Number 5,858,937 (Richards et al.), and available under the trade name Dequest® (Montsanto Co., St. Louis, Missouri).

Amphoteric surfactants suitable for use in a composition according to the present invention include materials of the type are offered commercially under the trade name Miranol^{™} (Noveon, Inc., Cleveland, Ohio). Another useful class of amphoteric surfactants is exemplified by cocoamidopropyl betaine, commercially available from various sources.

Various other ionic as well as amphoteric and anionic surfactants suitable for in the invention can be readily ascertained, in view of the foregoing description, from McCutcheon's Detergents and Emulsifiers, North American Edition, McCutcheon Division, MC Publishing Co., Glen Rock, NJ 07452 and the CTFA International Cosmetic Ingredient Handbook, Published by The Cosmetic, Toiletry, and Fragrance Association, Washington, D.C.

Preferably, the surfactants, when present, are employed in a total amount from about 0.01 to about 15 weight percent, preferably about 0.1 to about 9.0 weight percent, and most preferably about 0.1 to about 7.0 weight percent.

As an illustration of the present invention, several examples are provided below. These examples serve only to further illustrate aspects of the invention and should not be construed as limiting the invention.

### EXAMPLE 1 - Preparation of Test Solutions:

Sample solutions for testing were prepared in accordance with the formulations set forth below in Table 1.

**TABLE 1**

| **TEST SOLUTIONS** | | | | | |
|---|---|---|---|---|---|
| **Ingredients %W/W** | **Test Solution** | | | | |
| | **1** | **2** | **3** | **4** | **5** |
| Pluronic P123 | 0.250 | 0.500 | 1.000 | 2.500 | 5.000 |
| Tetronic 1107 | 1.00 | 1.00 | 1.00 | 1.00 | 1.00 |
| Sodium Borate | 0.09 | 0.09 | 0.09 | 0.09 | 0.09 |
| Boric Acid | 0.64 | 0.64 | 0.64 | 0.64 | 0.64 |
| EDTA | 0.11 | 0.11 | 0.11 | 0.11 | 0.11 |
| PHMB (ppm) | 1.0 | 1.0 | 1.0 | 1.0 | 1.0 |
| Dequest 2016 | 0.03 | 0.03 | 0.03 | 0.03 | 0.03 |
| Sodium Chloride | 0.49 | 0.49 | 0.49 | 0.49 | 0.49 |
| Purified Water | Q.S. to 100 gm | | | | |

### EXAMPLE 2 - Preparation of Artificial Tears for Lipid Cleaning (Model 1 Hands-Off Regimen):

Artificial tears for use in testing were prepared in accordance with the formulation set forth below in Table 2. The pH of the artificial tears was adjusted to 7.2 using 1 N HCl. Osmolarity = 320 mOsm/kg.

**TABLE 2**

| **Artificial Tears Formulation** | |
|---|---|
| **Ingredients** | **%W/W** |
| Salts and Buffer | |
| NaCl | 0.7 |
| KCl | 0.2 |
| NaHCO₃ | 0.12 |
| CaCO₃ | 0.01 |
| NaH₂PO₄ H₂O | 0.01 |
| 3-(N-morpholino)propanesulfonic acid | 0.4 |
| | |

| Lipids | |
|---|---|
| Palmitic acid methyl ester | 0.25 |
| Cholesterol | 0.25 |
| Squalene | 0.25 |
| | |

| Proteins | |
|---|---|
| Mucin | 0.01 |
| Lactoferrin | 0.01 |
| Human albumin serum | 0.01 |
| Lysozyme | 0.25 |

### EXAMPLE 3 - Preparation of Artificial Tears for Lipid Cleaning (Model 2 Hands-Off Regimen):

Super sterol ester (9.9 gm) was heated slowly until it melted. After melting, 0.1 gm of Sudan I was added to form a 99:1 mixture. The same was then mixed until the solution was homogenous. Using a glass pipette, five drops of mixture were transferred into each 12 cm x 12 mm glass screw top test tube. The test tubes were visually checked to ensure that the volume at the bottom of each test tube were of about equal size and diameter. Each batch should accommodate 40 to 50 tubes.

After preparing the tubes, 5 mL of the test solution was placed into each prepared tube. The tubes were then placed in a rotary shaker for 24 hours on 150 revolutions per minute (rpm) and 25 °C. After cleaning, the absorbance (ABS) was measured at 485.5 nm.

### EXAMPLE 4 - Lipid Cleaning Results from Model 2 Hands-Off Regimen:

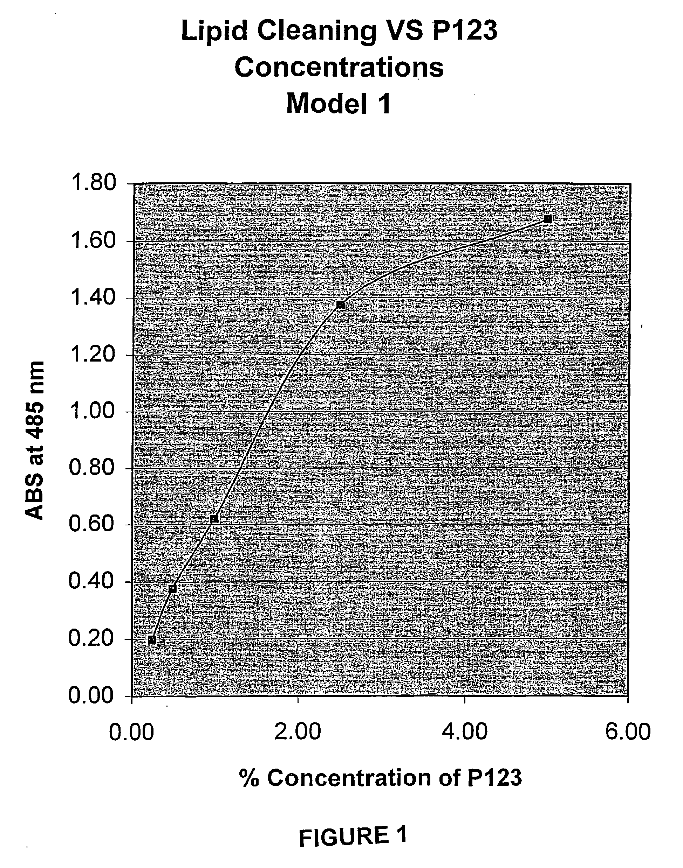

The lipid cleaning results from the Model 2 Hands-Off Regimen using SureVue^{™} (Bausch & Lomb Incorporated, Rochester, New York) lenses are set forth below in Table 3 and illustrated in Figure 1.

**TABLE 3**

| **Model 2 Hands-Off Regimen using SureVue Contact Lenses** | |
|---|---|
| **Test Solution** | **Average ABS (485.5 nm)** |
| 1 | 0.198 |
| 2 | 0.376 |
| 3 | 0.620 |
| 4 | 1.376 |
| 5 | 1.675 |

### EXAMPLE 5 - Effect of Solution Volumes on the Lipid Cleaning Efficacy from Model 2 Hands-Off Regimen:

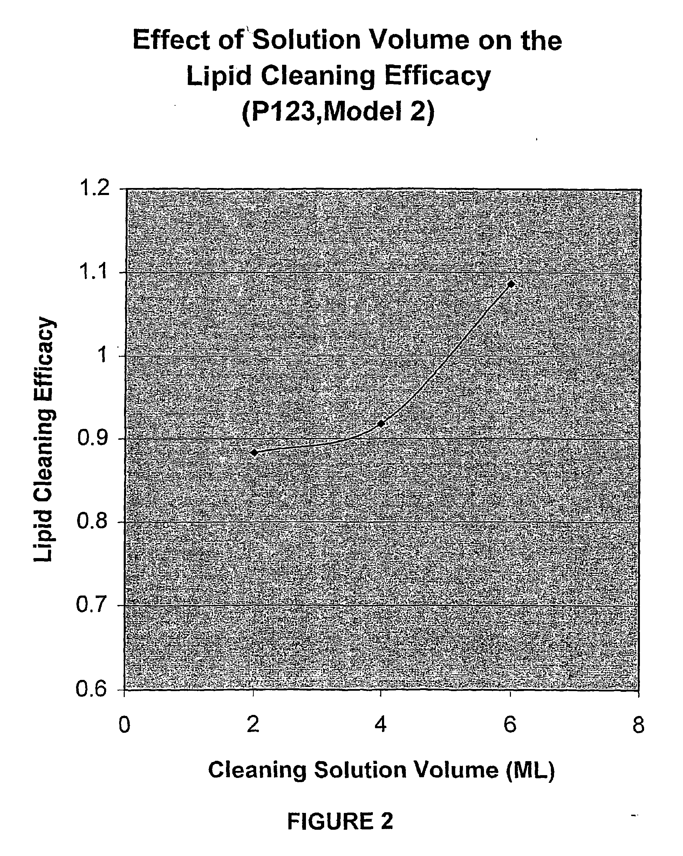

The effect of solution volumes on lipid cleaning efficacy from the Model 2 Hands-Off Regimen using SureVue^{™} lenses are set forth below in Table 4 and illustrated in Figure 2.

**TABLE 4**

| **Model 2 Hands-Off Regimen using SureVue Contact Lenses** | |
|---|---|
| **Test Solution** | **Average Absorbance** |
| **Volume (mL)** | **(485.5 nm)** |
| 2.0 | 0.883 |
| 4.0 | 0.918 |
| 6.0 | 1.086 |

### EXAMPLE 6 - Lipid Cleaning Results from Model 1 Hands-Off Regimen:

The lipid cleaning results from the Model 1 Hands-Off Regimen using PureVision^{™} (Bausch & Lomb Incorporated) lenses are set forth below in Table 5.

**TABLE 5**

| **Model 1 Hands-Off Regimen using PureVision Contact Lenses** | |
|---|---|
| **Test Solutions** | **Lipid Cleaning Efficacies (%)** |
| Control | 70.5 |
| Control + Pluronic P123 | 95.7 |

Compositions of the present invention may be used for soaking a contact lens whereby the aqueous composition comprises one or more nonionic polyether surfactants having a HLB less than 12 and a nonionic surfactant having a HLB value of 18 or greater in an amount effective to reduce the formation of lipid deposits on the contact lens.

Compositions of the present invention may also be used for rinsing or soaking a contact lens whereby the aqueous composition comprises one or more nonionic polyether surfactants having a HLB less than 12 and a nanionic surfactant having a HLB value of 18 or greater in an amount effective to remove lipid deposits from surfaces of the contact lens.

Still another method of using compositions of the present invention comprises preventing deposition of lipids on a contact lens while worn on the eye. This method comprises soaking the contact lens in an aqueous composition with one or more nonionic polyether surfactants having a HLB less than 12 and a nonionic surfactant having a HLB value of 18 or greater present in an effective amount, and inserting the contact lens in the eye without rinsing the composition from the contact lens, or instilling one or more drops of the composition in the eye while wearing the contact lens, to prevent deposition of lipids on a contact lens while worn in the eye.

Although various preferred embodiments have been illustrated, many other modifications and variations of the present invention are possible to the skilled practitioner. It is therefore understood that, within the scope of the claims, the present invention can be practiced other than as herein specifically described.

## Claims

1. A composition for reducing the amount of lipid deposits on a contact lens comprising:
one or more nonionic polyether surfactants having a HLB value of less than 12;
a nonionic surfactant having a HLB value of 18 or greater; and
one or more antimicrobial agents.

2. The composition of any of the previous claims, wherein the composition further comprises at least one member selected from the group consisting of a buffering agent, a chelating agent and an osmolarity adjusting agent.

3. The composition of claim 1, wherein said one or more antimicrobial agents are present in an amount effective to disinfect the contact lens.

4. The composition of claim 1, wherein the composition comprises about 0.1 to about 6.0 weight percent of said nonionic polyether surfactant and about 0.05 to about 0.5 weight percent of said antimicrobial agent.

5. The composition of claim 1, wherein the composition further comprises a chelating agent and a buffering agent selected from the group consisting of borate buffers, phosphate buffers and citrate buffers.

6. The composition of claim 1, wherein said one or more antimicrobial agents include [4-tris(2-hydroxyethyl)ammonio]-2-butenyl-ca-[tris(2-hydroxyethyl)ammonio]dichloride and poly(hexamethylene biguanide).

7. The composition of claim 1, wherein the non-ionic surfactant having a HLB value of 18 or greater is a poloxamer or a polaxamine.

8. The composition of claims 1, wherein the composition further comprises propylene glycol as a wetting agent.

9. A method of removing lipid deposits from the surfaces of a contact lens, or preventing deposition of lipids on said lens, the method comprising:
soaking said contact lens in an aqueous composition that comprises one or more nonionic polyether surfactants having a HLB value of less than 12 and a nonionic surfactant having a HLB value of 18 or greater in an amount effective to remove or prevent lipid deposits from surfaces of said contact lens, said composition further comprising an antimicrobial agent in an amount effective to disinfect the contact lens; and
inserting said contact lens in an eye with or without rinsing said composition from said contact lens.

10. The method of claim 9, wherein the composition further comprises at least one member selected from the group consisting of an antimicrobial agent, a buffering agent, a chelating agent, and an osmolarity adjusting agent.

11. The method of claim 9, wherein the composition comprises 0.05 to 0.5 weight percent of said antimicrobial agent.

12. The method of claim 11, wherein the composition further comprises a chelating agent and a buffering agent selected from the group consisting of borate buffers, phosphate buffers and citrate buffers.

13. A method of cleaning a contact lens comprising:
soaking the contact lens in an aqueous composition that comprises one or more nonionic polyether surfactants having a HLB value of less than 12 and a nonionic surfactant having a HLB value of 18 or greater in an amount effective to remove lipids on the contact lens, said composition further comprising an antimicrobial agent in an amount effective to disinfect the contact lens; and
rinsing the contact lens to remove the lipids.

14. The method of claim 13, wherein the lipids are removed without manual rubbing.

15. The method of claim 13, wherein the contact lens is rinsed with said composition and then is inserted directly into the eye.

16. The method of claim 13, wherein the antimicrobial agent is present in an amount of from 0.05 to 0.5 weight percent to disinfect the contact lens.

17. The method of claims 9 or 13, wherein said one or more antimicrobial agents include [4-tris(2-hydroxyethyl)ammonio]-2-butenyl-ω-[tris(2-hydroxyethyl)ammonio]dichloride and poly(hexamethylene biguanide).

18. The method of claims 9, 13 or 17, wherein said contact lens is a soft, silicon hydrogel lens.

## Patentansprüche

1. Zusammensetzung zum Reduzieren der Menge an Lipid-Ablagerungen auf einer Kontaktlinse, umfassend:
ein oder mehrere nichtionische(s) Polyether-Tensid(e), das/die einen HLB-Wert von weniger als 12 aufweist/aufweisen; ein nichtionisches Tensid, dass einen HLB-Wert von 18 oder größer aufweist; und ein oder mehrere antimikrobielle(s) Mittel.

2. Zusammensetzung gemäß dem vorherigen Anspruch, wobei die Zusammensetzung ferner mindestens ein Element ausgewählt aus der Gruppe bestehend aus einer Puffersubstanz, einem chelatbildenden Mittel und einem Mittel zum Einstellen der Osmolarität umfasst.

3. Zusammensetzung gemäß Anspruch 1, wobei das eine oder mehrere antimikrobielle(n) Mittel in einer Menge vorliegt/vorliegen, die wirksam ist, um die Kontaktlinse zu desinfizieren.

4. Zusammensetzung gemäß Anspruch 1, wobei die Zusammensetzung etwa 0.1 bis etwa 6.0 Gew.-% des nichtionischen Polyether-Tensids und etwa 0.05 bis etwa 0.5 Gew.-% des antimikrobiellen Mittels umfasst.

5. Zusammensetzung gemäß Anspruch 1, wobei die Zusammensetzung ferner ein chelatierendes Mittel und eine Puffersubstanz, ausgewählt aus der Gruppe bestehend aus Boratpuffer, Phosphatpuffer und Citratpuffer umfasst.

6. Zusammensetzung gemäß Anspruch 1, wobei das eine oder mehrere antimikrobielle(n) Mittel [4-Tris(2-hydroxyethyl)ammonium]-2-butenyl-ω-[tris(2-hydroxyethyl)ammonium]-dichlorid und Poly(hexamethylenbiguanid) beinhalten.

7. Zusammensetzung gemäß Anspruch 1, wobei das nichtionische Tensid, das einen HLB-Wert von 18 oder größer aufweist, ein Poloxamer oder Poloxamin darstellt.

8. Zusammensetzung gemäß Anspruch 1, wobei die Zusammensetzung ferner Propylenglykol als ein Benetzungsmittel umfasst.

9. Verfahren zum Entfernen von Lipid-Ablagerungen von der Oberfläche einer Kontaktlinse, oder Verhindern der Ablagerung von Lipiden auf der Linse, wobei das Verfahren umfasst:
Einweichen der Kontaktlinse in einer wässrigen Zusammensetzung, die ein oder mehrere nichtionische(s) Polyether-Tensid(e) umfasst, das/die einen HLB-Wert von weniger als 12 aufweist/aufweisen und ein nichtionisches Tensid, das einen HLB-Wert von 18 oder größer aufweist, in einer Menge, die wirksam ist, um Lipidablagerungen auf der Oberfläche der Kontaktlinse zu entfernen oder zu verhindern, wobei die Zusammensetzung ferner ein antimikrobielles Mittel in einer Menge umfasst, die wirksam ist, um die Kontaktlinse zu desinfizieren; und
Einsetzen der Kontaktlinse in ein Auge mit oder ohne Abspülen der Zusammensetzung von der Kontaktlinse.

10. Verfahren gemäß Anspruch 9, wobei die Zusammensetzung ferner mindestens ein Element ausgewählt aus der Gruppe bestehend aus einem antimikrobiellen Mittel, einer Puffersubstanz, einem chelatbildenden Mittel und einem Mittel zum Einstellen der Osmolarität umfasst.

11. Verfahren gemäß Anspruch 9, wobei die Zusammensetzung 0.05 bis 0.5 Gew.-% des antimikrobiellen Mittels umfasst.

12. Verfahren gemäß Anspruch 11, wobei die Zusammensetzung ferner ein chelatbildendes Mittel und eine Puffersubstanz ausgewählt aus der Gruppe bestehend aus Boratpuffer, Phosphatpuffer und Citratpuffer umfasst.

13. Verfahren zum Reinigen einer Kontaktlinse, umfassend:
Einweichen der Kontaktlinse in einer wässrigen Zusammensetzung, die ein oder mehrere nichtionische Polyether-Tensid(e) umfasst, das/die einen HLB-Wert von weniger als 12 aufweist/aufweisen und ein nichtionisches Tensid, das einen HLB-Wert von 18 oder größer aufweist, in einer Menge, die wirksam ist um Lipidablagerungen auf der Kontaktlinse zu entfernen, wobei die Zusammensetzung ferner ein antimikrobielles Mittel in einer Menge umfasst, die wirksam ist, um die Kontaktlinse zu desinfizieren; und
Abspülen der Kontaktlinse um die Lipide zu entfernen.

14. Verfahren gemäß Anspruch 13, wobei die Lipide ohne manuelles Reiben entfernt werden.

15. Verfahren gemäß Anspruch 13, wobei die Kontaktlinse mit der Zusammensetzung abgespült und dann direkt in das Auge eingesetzt wird.

16. Verfahren gemäß Anspruch 13, wobei das antimikrobielle Mittel in einer Menge von etwa 0.05 bis 0.5 Gew.-% vorliegt, um die Kontaktlinse zu desinfizieren.

17. Verfahren gemäß der Ansprüche 9 oder 13, wobei das eine oder mehrere antimokrobielle(n) Mittel [4-Tris(2-hydroxyethyl)ammonium]-2-butenyl-ω-[tris(2-hydroxyethyl)ammonium]-dichlorid und Poly(hexamethylenbiguanid) beinhaltet/beinhalten.

18. Verfahren gemäß der Ansprüche 9, 13 oder 17, wobei die Kontaktlinse eine weiche Silikonhydrogellinse darstellt.

## Revendications

1. Composition pour la réduction de la quantité de dépôts lipidiques sur une lentille de contact comprenant
un ou plusieurs tensioactifs de type polyéther non ioniques ayant une valeur HLB (balance hydrophile lipophile) de moins de 12 ;
un tensioactif non ionique ayant une valeur HLB de 18 ou plus ; et
un ou plusieurs agents antimicrobiens.

2. Composition selon la revendication 1, dans laquelle la composition comprend en outre au moins un élément choisi dans le groupe constitué par un agent tampon, un agent chélatant et un agent d'ajustement de l'osmolarité.

3. Composition selon la revendication 1, dans laquelle un ou plusieurs agents antimicrobiens sont présents en une quantité efficace pour désinfecter la lentille de contact.

4. Composition selon la revendication 1, dans laquelle la composition comprend environ 0,1 à environ 6,0 % en poids dudit tensioactif de type polyéther non ionique et d'environ 0,05 à environ 0,5 % en poids dudit agent antimicrobien.

5. Composition selon la revendication 1, dans laquelle la composition comprend en outre un agent chélatant et un agent tampon choisi dans le groupe constitué par les tampons de borate, les tampons de phosphate et les tampons de citrate.

6. Composition selon la revendication 1, dans laquelle ledit ou lesdits agents antimicrobiens comprennent le dichlorure de [4-tris(2-hydroxy-éthyl)ammonio]-2-butényl-ω-[tris(2-hydroxyéthyl)ammonio] et le poly(hexaméthylène biguanide).

7. Composition selon la revendication 1, dans laquelle le tensioactif non ionique ayant une valeur HLB de 18 ou plus est un poloxamère ou une polaxamine.

8. Composition selon la revendication 1, dans laquelle la composition comprend en outre le propylène glycol en tant qu'agent mouillant.

9. Procédé d'élimination de dépôts lipidiques sur les surfaces d'une lentille de contact, ou de prévention de dépôt de lipides sur ladite lentille, le procédé comprenant :
l'immersion de ladite lentille de contact dans une composition aqueuse qui comprend un ou plusieurs tensioactifs de type polyéther non ioniques ayant une valeur HLB de moins de 12 et un tensioactif non ionique ayant une valeur HLB de 18 ou plus en une quantité efficace pour éliminer ou empêcher les dépôts lipidiques sur les surfaces de ladite lentille de contact, ladite composition comprenant en outre un agent antimicrobien en une quantité efficace pour désinfecter la lentille de contact ; et
l'insertion de ladite lentille de contact dans un oeil avec ou sans rinçage de ladite composition de ladite lentille de contact.

10. Procédé selon la revendication 9, dans lequel la composition comprend en outre au moins un élément choisi dans le groupe constitué par un agent antimicrobien, un agent tampon, un agent chélatant, et un agent d'ajustement de l'osmolarité.

11. Procédé selon la revendication 9, dans lequel la composition comprend de 0,05 à 0,5 % en poids dudit agent antimicrobien.

12. Procédé selon la revendication 11, dans lequel la composition comprend en outre un agent chélatant et un agent tampon choisi dans le groupe constitué par les tampons de borate, les tampons de phosphate et les tampons de citrate.

13. Procédé de nettoyage d'une lentille de contact comprenant :
l'immersion de la lentille de contact dans une composition aqueuse qui comprend un ou plusieurs tensioactifs de type polyéther non ioniques ayant une valeur HLB de moins de 12 et un tensioactif non ionique ayant une valeur HLB de 18 ou plus en une quantité efficace pour éliminer les lipides sur la lentille de contact, ladite composition comprenant en outre un agent antimicrobien en une quantité efficace pour désinfecter la lentille de contact ; et
le rinçage de la lentille de contact pour éliminer les lipides.

14. Procédé selon la revendication 13, dans lequel les lipides sont éliminés sans frottement manuel.

15. Procédé selon la revendication 13, dans lequel la lentille de contact est rincée avec ladite composition, puis insérée directement dans l'oeil.

16. Procédé selon la revendication 13, dans lequel l'agent antimicrobien est présent en une quantité de 0,05 à 0,5 % en poids pour désinfecter la lentille de contact.

17. Procédé selon la revendication 9 ou 13, dans lequel ledit ou lesdits agents antimicrobiens comprennent le dichlorure de [4-tris(2-hydroxy-éthyl)ammonio]-2-butényl-ω-[tris(2-hydroxyéthyl)ammonio] et le poly(hexaméthylène biguanide).

18. Procédé selon la revendication 9, 13 ou 17, dans lequel ladite lentille de contact est une lentille en hydrogel de silicone souple.
